# EUROPÄISCHE PATENTSCHRIFT

(11) **EP 2 608 745 B1**
(45) Veröffentlichungstag und Bekanntmachung des Hinweises auf die Patenterteilung: **06.07.2016**
(21) Anmeldenummer: 11748879.1
(22) Anmeldetag: 23.08.2011
(51) Int. Cl.: A61B 17/70, A61B 17/68, A61B 17/84

(54) **ANKERHÜLSE**
ANCHOR SLEEVE
DOUILLE D'ANCRAGE

(30) Priorität: 25.08.2010 CH 13642010
(43) Veröffentlichungstag der Anmeldung: 03.07.2013
(73) Patentinhaber: Schefer, Arnold, 4616 Kappel (CH)
(72) Erfinder: Schefer, Arnold, 4616 Kappel (CH)
(74) Vertreter: Wagner, Wolfgang Heribert
(86) Internationale Anmeldenummer: PCT/CH2011/000191
(87) Internationale Veröffentlichungsnummer: WO 2012/024806

(56) Entgegenhaltungen:
- EP-A2- 1 491 162
- WO-A1-2008/154762
- WO-A1-2009/132445
- WO-A2-01/70135
- WO-A2-2009/132472
- DE-A1- 19 741 087
- DE-C1- 19 634 698
- FR-A1- 2 629 337
- US-A- 4 888 024
- US-A1- 2009 099 609

## Beschreibung

### Technisches Gebiet

Die Erfindung betrifft eine Ankerhülse zur Verbindung mit mindestens einem Knochen, insbesondere zur Verbindung mit zwei Bruchstücken eines Knochens zum Festhalten derselben in einer bestimmten gegenseitigen Lage.

### Stand der Technik

Es sind zahlreiche Anker bekannt, welche zur Befestigung etwa einer Platte an einem Knochen oder einem Bruchstück eines Knochens in einem Bohrloch in dem letzteren verankert werden können.

So zeigt US 7 338 493 B1 einen Anker, welcher eine als Stutzen ausgebildete Ankerhülse umfasst, mit an einem proximalen Ende einem Aussengewinde und einem vom proximalen zu einem distalen Ende durchgehenden oder knapp vor dem letzteren verschlossenen Verteilkanal, der an einem an das proximale Ende anschliessenden Verankerungsabschnitt ein Innengewinde zum Eingriff mit einem Gewinde eines Ankerbolzens aufweist und an einem anschliessenden Restabschnitt mit seitlichen Austrittsöffnungen versehen ist. Nach dem Einsetzen der Ankerhülse in das als Sackloch ausgebildete Bohrloch wird vom proximalen Ende durch den Verteilkanal als Füllmasse Knochenzement eingeleitet, welcher durch die Austrittsöffnungen in das Bohrloch austritt und dasselbe ausfüllt.

Diese Ausführung hat jedoch den Nachteil, dass vor allem bei geringer Viskosität desselben Knochenzement aus dem Bohrloch ausfliessen kann, da es gewöhnlich einige Minuten dauert, bis er ausgehärtet ist. Wenn das Bohrloch nicht als Sackloch, sondern durchgehend ausgebildet ist, ist ein Ausfliessen von Knochenzement am distalen Ende desselben kaum zu vermeiden. Der Anker eignet sich daher im allgemeinen nicht zur direkten Verbindung von zwei Bruchstücken eines Knochens.

Ein Anker, welcher eine gattungsgemässe Ankerhülse mit einem Stutzen mit Austrittsöffnungen für ein Füllmaterial und einer denselben umgebenden Hülle umfasst, ist aus US 2009/099 609 A1 bekannt. Die Austrittsöffnungen sind dabei durchwegs im Bereich des distalen Endes des Stutzens angeordnet. Die Verankerung der Hülle ist daher auf einen verhältnismässig engen Bereich konzentriert, sodass die Gefahr eines Ausbrechens des Knochens weiter besteht und eine Ausweitung des Bohrlochs im Endbereich nötig ist, die dann von der durch Füllstoff aufgeblähten Hülle ausgefüllt wird.

### Darstellung der Erfindung

Der Erfindung liegt die Aufgabe zu Grunde, eine gattungsgemässe Ankerhülse insofern zu verbessern, als der Kontakt mit dem Knochen über eine grosse Fläche verteilt wird. Diese Aufgabe wird durch die Merkmale im Kennzeichen des Anspruchs 1 erfüllt.

Die erfindungsgemässe Ankerhülse ist über eine grössere Länge mit dem Knochen in Kontakt, sodass die lokale Belastung des Knochens und damit verbundene Risiken des Ausbrechens deutlich geringer sind.

### Kurze Beschreibung der Zeichnungen

Im folgenden wird die Erfindung anhand von Figuren, welche lediglich Ausführungsbeispiele darstellen, näher erläutert.

Es zeigen
- Fig. 1a: einen axialen Längsschnitt durch eine erfindungsgemässe Ankerhülse gemäss einer ersten Ausführungsform, in Bohrlöchern eingesetzt,
- Fig. 1b: einen Querschnitt durch die Ankerhülse längs B-B in Fig. 1a,
- Fig. 2: einen Längsschnitt entsprechend Fig. 1a am Ende des Auffüllens der Ankerhülse und der Bohrlöcher mit Füllmasse,
- Fig. 3: einen axialen Längsschnitt durch eine erfindungsgemässe Ankerhülse gemäss einer zweiten Ausführungsform, in Bohrlöchern eingesetzt, und
- Fig. 4: einen Längsschnitt entsprechend Fig. 3 am Ende des Auffüllens der Ankerhülse mit Füllmasse.

### Wege zur Ausführung der Erfindung

Gemäss einer ersten Ausführungsform weist die Ankerhülse einen Stutzen 1 auf, welcher einen von einer Einfüllöffnung 2 an seinem proximalen Ende ausgehenden Verteilkanal 3 umgibt. Der Stutzen 1, der vorzugsweise aus geringfügig elastischem Kunststoff besteht, weist im Bereich der Einfüllöffnung 2 einen nach aussen vorspringenden umlaufenden Kragen 4 mit gegen das distale Ende sich leicht konisch verengender Mantelfläche auf. Am distalen Ende ist der Stutzen 1 offen, sodass er dort eine Endöffnung 5 bildet. Von dieser gehen axiale Schlitze 6 aus, welche wie die Endöffnung 5, an welche sie anschliessen, Austrittsöffnungen bilden, die sich über den grösseren Teil der Länge des Stutzens 1 erstrecken. In der Nähe des proximalen Endes des Stutzens 1 ist eine sackartige geschlossene Hülle 7 aus flexiblem Material an seiner Aussenseite anliegend befestigt, indem ihr Rand durch einen elastischen Ring 8, z.B. einen Sprengring aus Federstahl oder einen geschlossenen Ring aus elastischem Kunststoff, in einer unmittelbar distal des Kragens 4 umlaufenden Rille lösbar festgehalten wird. Die Hülle 7 umschliesst den Abschnitt der Aussenseite des Stutzens 1, welcher die Austrittsöffnungen, d.h. die Endöffnung 5 und diejenigen, welche von den Schlitzen 6 gebildet werden, trägt. Sie ist dabei wesentlich länger als dieser, sodass sie über sein distales Ende beträchtlich hinausgeht und besteht aus inelastischem Material, z.B. aus einem verhältnismässig lockeren Gewebe oder Gewirk aus Kunststoff oder mineralischer Faser oder wie dargestellt aus gelochter Folie aus Kunststoff und ist daher begrenzt durchlässig.

Die Ankerhülse verbindet zwei Bruchstücke 9, 10 eines Knochens, in denen fluchtende Bohrlöcher 11, 12 angebracht sind, welche die Ankerhülse aufnehmen. Das erste Bruchstück 9 besteht z.T. aus harter Rinde 9' und z.T. aus weicher, poröser Spongiosa 9" und das zweite Bruchstück nur aus Spongiosa 10". Der Stutzen 1 ist etwas in das Bohrloch 11 im ersten Bruchstück 9 eingedrückt, sodass der äussere Rand des Kragens 4 fest an der an den Rand des Bohrlochs 11 im ersten Bruchstück 9 anschliessenden Innenfläche desselben anliegt und dort elastisch gegen die Rinde 9' drückt. Der verbleibende Teil des Stutzens 1 ist dagegen durch einen umlaufenden Spalt von der Wand des Bohrlochs 11 getrennt.

Die Hülle 7 reicht bis zum Grund des Bohrlochs 12 im zweiten Bruchstück 10, das als Sackloch ausgebildet ist.

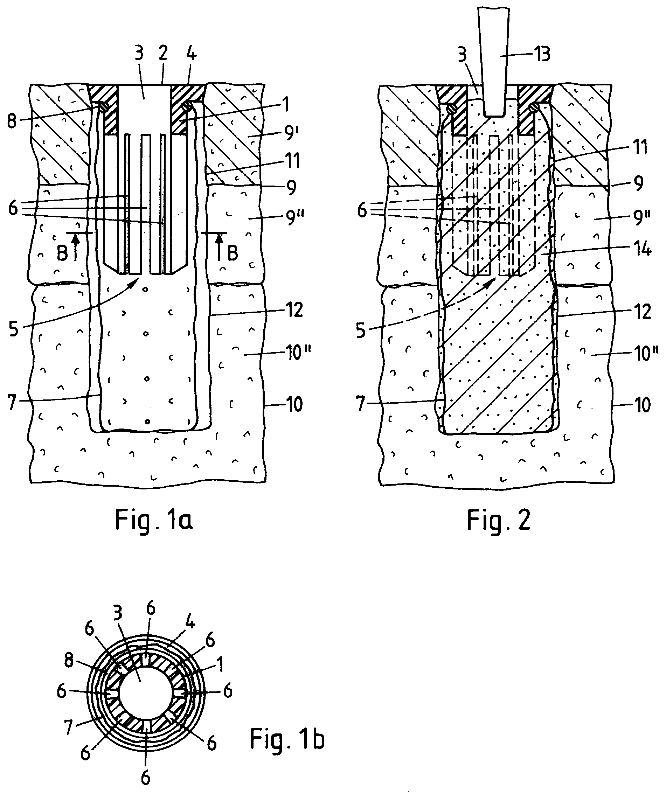

Nach dem Einsetzen der Ankerhülse in die Bohrlöcher 11, 12 wird mittels einer Hohlnadel 13 (Fig. 2) durch die Einfüllöffnung 2 eine aushärtbare Füllmasse 14, in der Regel Knochenzement, in den Verteilkanal 3 eingeleitet. Sie dringt durch die Austrittsöffnungen, d.h. die Endöffnung 5 und die Schlitze 6 nach aussen und füllt so die Hülle 7 auf. Dabei tritt ein verhältnismässig geringer Teil der Füllmasse 14 durch die Hülle 7 aus und füllt so die Bohrlöcher 11, 12 vollständig aus. Die Hohlnadel 13 wird daraufhin zurückgezogen und die Füllmasse 14 härtet in einigen Minuten aus.

Die beiden Bruchstücke 9, 10 sind durch den ausgehärteten Knochenzement und die darin eingebettete Hülle 7 fest und zuverlässig verbunden. Abgesehen von einer Verstärkung der Füllmasse 14, die vor allem dann einen wesentlichen Beitrag zur Festigkeit der Verbindung leisten kann, wenn die Hülle 7 aus einem Gewebe oder Gewirk aus fester mineralischer Faser besteht, erfüllt die Hülle 7 die Aufgabe, die Strömung der Füllmasse 14 in die Bohrlöcher 11, 12 zu lenken und insbesondere eine gleichmässige Verteilung derselben sicherzustellen. Z.B. wird ein Austreten grösserer Mengen in den Spalt zwischen den Bruchstücken 9, 10 durch die Hülle 7 verhindert. Dabei kann die Struktur des Gewebes oder Gewirks oder die Lochung der Folie über die Ausdehnung, insbesondere die Länge der Hülle 7 variieren, sodass dem Austreten der Füllmasse 14 ein grösserer oder kleinerer Widerstand entgegensteht. Wenn sie so geeignet gelenkt wird, füllt die Füllmasse 14 die Bohrlöcher 11, 12 satt aus und geht mit dem Knochen eine innige Verbindung ein, die etwa durch teilweises Eindringen vor allem in die Spongiosa 9", 10" breit abgestützt sein kann, sodass ein Ausbrechen nicht zu befürchten ist.

Der Stutzen kann statt aus Kunststoff auch aus Keramik oder Metall bestehen.

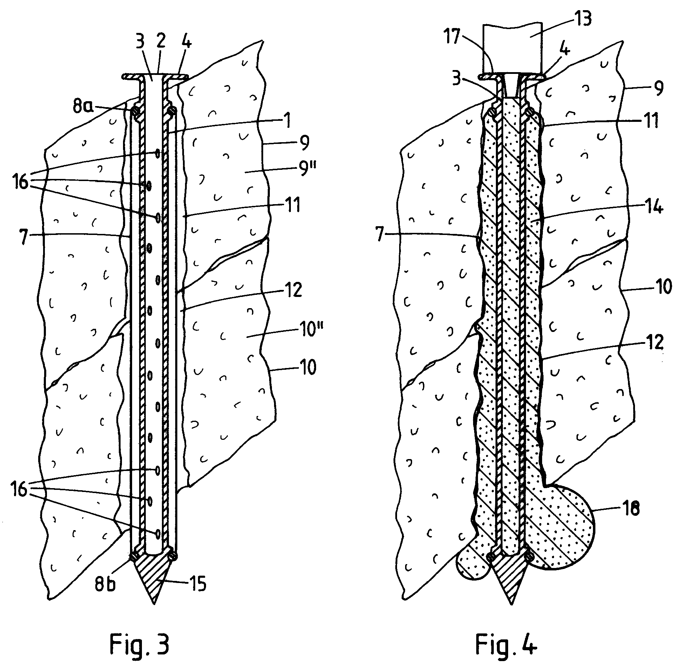

Gemäss einer zweiten Ausführungsform (Fig. 3) weist die Ankerhülse ebenfalls einen Stutzen 1 auf, ebenfalls mit am proximalen Ende einer Einfüllöffnung 2, an welche ein Verteilkanal 3 anschliesst sowie einem nach aussen abstehenden umlaufenden Kragen 4. Der Stutzen 1 besteht vorzugsweise aus Metall, z.B. aus Titan oder rostfreiem Stahl. Der Verteilkanal 3 geht bis nahe an das distale Ende des Stutzens 1 durch und ist dort verschlossen, während der Stutzen in eine Spitze 15 ausläuft. Der Verteilkanal 3 ist durch runde oder schlitzförmige Löcher 16 mit über dessen Länge verteilten Austrittsöffnungen an der Aussenseite des Stutzens 1 verbunden.

An dieser Aussenseite ist wiederum eine denselben umgebende Hülle 7 angeordnet. Sie ist schlauchartig ausgebildet und umschliesst den Stutzen 1 sowohl knapp distal des Kragens 4 als auch knapp proximal der Spitze 15, wo ihr proximaler bzw. distaler umlaufender Rand jeweils durch einen elastischen Ring 8a;b in einer Rille lösbar festgehalten wird. Die Hülle 7 umschliesst so alle Austrittsöffnungen. Die Hülle 7 ist in diesem Fall als undurchlässige Folie aus elastischem Material, vorzugsweise Kunststoff, ausgebildet. Die Ankerhülse ist wiederum in Bohrlöcher 11, 12 eingesetzt, welche an Bruchstücken 9, 10, die hier beide aus Spongiosa 9", 10" bestehen, angebracht wurden und hier beide durchgehend ausgebildet sind, wobei die Ankerhülse mit ihrem distalen Ende etwas aus dem Bohrloch 12 herausragt. Die Spitze 15 erleichtert das Einführen der Ankerhülse in die Bohrlöcher 9, 10.

Beim Einleiten von Füllmasse 14 (Fig. 4) wird vorzugsweise eine Hohlnadel 13 eingesetzt, welche mit einem umlaufenden Absatz 17, der einen Dichtungsring tragen kann, gegen die proximale Seite des Kragens 4 drückt, sodass ein leichter Ueberdruck in der Füllmasse 14 erzeugt werden kann. Die elastische Hülle 7 wird dann durch die Füllmasse 14, welche durch die Löcher 16 aus dem Verteilkanal 3 autritt, gegen die Wände der Bohrlöcher 11, 12 gedrückt, sodass sie sich im wesentlichen an deren Unebenheiten anpasst und insbesondere Vertiefungen ganz oder teilweise ausfüllt. Am distalen Ende wird sie etwas aufgebläht und bildet einen Wulst 18, dessen Durchmesser grösser ist als der der Bohrlöcher 11, 12. Nach dem Aushärten der Füllmasse 14 wird die Hohlnadel 13 zurückgezogen. Die Ankerhülse ist in den Bohrlöchern 11, 12 grossflächig verankert und verbindet die Bruchstücke 9, 10 zuverlässig, ohne dass der Knochen mit der Füllmasse 14 direkt in Berührung käme. Durch das Anliegen des Kragens 4 an der Aussenseite des Bruchstücks 9 und des Wulstes 18 an der entgegengesetzten Aussenseite des Bruchstücks 10 sind die Bruchstücke 9, 10 ausserdem verspannt, was die Zuverlässigkeit der Verbindung erhöht.

Es sind viele Abwandlungen möglich, ohne dass der Bereich der Erfindung verlassen würde. Der Stutzen kann etwa aus mehr oder weniger hartem Kunststoff, Metall, z.B. rostfreiem Stahl oder Titan oder einer geeigneten Legierung oder aus Keramik bestehen. Er kann, vor allem wenn er aus hartem Material besteht, etwa im Bereich seines proximalen Endes mit einem Aussengewinde versehen sein, das ein Eindrehen desselben in ein Bohrloch ermöglicht, oder mit Längsrillen, die eine bessere Verankerung bei einem Einpressen oder -schlagen gewährleisten, so dass er für die Einleitung der Füllmasse und das Aushärten ausreichend verankerbar und keine weitere Fixierung erforderlich ist.

Obwohl sich die erfindungsgemässen Ankerhülsen besonders gut als Verbindungsanker zur Verbindung von Bruchstücken von Knochen eignen, können sie auch zur Verankerung z.B. von Platten o.dgl. an einem Knochen oder einem Bruchstück eines Knochens eingesetzt werden. Dazu kann etwa im Verteilkanal, insbesondere anschliessend an dessen proximales Ende, ein Innengewinde angebracht sein, welches ein Einschrauben eines entsprechenden Ankerbolzens gestattet.

Die flexible Hülle kann sackartig oder schlauchartig ausgebildet sein oder aus mehreren sackartig oder schlauchartig ausgebildeten Teilen bestehen, entscheidend ist, dass sie jene Teile der Aussenfläche des Stutzens umschliesst, welche Austrittsöffnungen tragen. Sie kann daneben elastisch oder inelastisch, schlaff oder halbsteif, für Füllmasse durchlässig oder undurchlässig ausgebildet sein oder über ihre Ausdehnung variierende mechanische Eigenschaften und Durchlässigkeit haben. Sie kann aus einem Gewebe oder Gewirk bestehen, das aus Kunstfaser oder auch ganz oder teilweise aus mineralischer Faser, z.B. Glasfaser oder Keramikfaser hergestellt ist. Sie kann auch ganz oder teilweise aus Kunststoffolie bestehen, welche durch Fasern verstärkt sein kann. Die Hülle kann auch fest mit dem Stutzen verbunden, z.B. mit ihm verklebt oder verschweisst sein, doch hat eine lösbare Befestigung den Vorteil, dass Stutzen und Hüllen z.T. frei kombiniert werden können.

Als Füllmasse kommen neben Knochenzement auch andere aushärtbare Massen in Frage, z.B. ein mit einem Härter versetztes Kunstharz.

### Bezugszeichenliste

- 1: Stutzen
- 2: Einfüllöffnung
- 3: Verteilkanal
- 4: Kragen
- 5: Endöffnung
- 6: Schlitze
- 7: Hülle
- 8: Ring
- 8a, b: Ringe
- 9: Bruchstück
- 9': Rinde
- 9": Spongiosa
- 10: Bruchstück
- 10": Spongiosa
- 11, 12: Bohrlöcher
- 13: Hohlnadel
- 14: Füllmasse
- 15: Spitze
- 16: Löcher
- 17: Absatz
- 18: Wulst

## Patentansprüche

1. Ankerhülse zur Verbindung mit mindestens einem Knochen oder Bruchstück (9, 10) eines Knochens, welche einen Stutzen (1) umfasst mit einem von einer Einlassöffnung (2) an einem proximalen Ende ausgehenden, in einer axialen Richtung gegen ein distales Ende sich erstreckenden Verteilkanal (3) mit mehreren an der Aussenseite des Stutzens (1) angeordneten mit dem Verteilkanal verbundenen Austrittsöffnungen sowie eine im Bereich seines proximalen Endes dicht an die Aussenseite des Stutzens (1) anschliessende, den die Austrittsöffnungen tragenden Teil der Aussenfläche des Stutzens umschliessende Hülle (7) aus flexiblem Material, **dadurch gekennzeichnet, dass** die Austrittsöffnungen sich über die Länge des Stutzens erstrecken oder über die Länge des Stutzens verteilt sind.

2. Ankerhülse nach Anspruch 1, **dadurch gekennzeichnet, dass** die Hülle (7) sackartig ausgebildet ist, derart, dass sie das distale Ende des Stutzens (1) umschliesst.

3. Ankerhülse nach Anspruch 2, **dadurch gekennzeichnet, dass** sich die Hülle (7) über das distale Ende des Stutzens (1) hinaus erstreckt.

4. Ankerhülse nach Anspruch 2 oder 3, **dadurch gekennzeichnet, dass** der Stutzen (1) an seinem distalen Ende eine Endöffnung (5) aufweist.

5. Ankerhülse nach Anspruch 1, **dadurch gekennzeichnet, dass** die Hülle (7) im Bereich ihres distalen Endes dicht an die Aussenseite des Stutzens (1) anschliesst.

6. Ankerhülse nach Anspruch 5, **dadurch gekennzeichnet, dass** der Stutzen (1) an seinem distalen Ende eine Spitze (15) aufweist.

7. Ankerhülse nach einem der Ansprüche 1 bis 6, **dadurch gekennzeichnet, dass** der Stutzen (1) aus Kunststoff, Keramik oder Metall wie Stahl oder Titan besteht.

8. Ankerhülse nach einem der Ansprüche 1 bis 7, **dadurch gekennzeichnet, dass** die Hülle (7) undurchlässig ist.

9. Ankerhülse nach einem der Ansprüche 1 bis 7, **dadurch gekennzeichnet, dass** die Hülle (7) über ihre Fläche verteilte Oeffnungen aufweist.

10. Ankerhülse nach einem der Ansprüche 1 bis 9, **dadurch gekennzeichnet, dass** die Hülle (7) aus inelastischem Material besteht.

11. Ankerhülse nach einem der Ansprüche 1 bis 9, **dadurch gekennzeichnet, dass** die Hülle (7) aus elastischem Material besteht.

12. Ankerhülse nach einem der Ansprüche 1 bis 11, **dadurch gekennzeichnet, dass** die Hülle (7) mindestens zum Teil aus Gewebe oder Gewirk besteht.

13. Ankerhülse nach einem der Ansprüche 1 bis 12, **dadurch gekennzeichnet, dass** die Hülle (7) mindestens zum Teil aus Folie besteht.

14. Ankerhülse nach einem der Ansprüche 1 bis 13, **dadurch gekennzeichnet, dass** die Hülle (7) mineralische Fasern enthält.

## Claims

1. An anchor sleeve for connection to at least a bone or a fragment (9, 10) of a bone, the anchor sleeve comprising a connecting piece (1) having a distribution channel (3) starting at an inlet opening (2) at a proximal end, extending in an axial direction towards a distal end and having multiple outlet openings distributed over the outside of the connecting piece (1), as well as a casing (7) made of a flexible material that sealingly connects to the outside of the connecting piece (1) in the area of its proximal end and encloses the part of the outside of the connecting piece that carries the outlet openings, **characterised in that** the outlet openings extend over the length of the connecting piece or are distributed over the. length of the connecting piece.

2. The anchor sleeve according to claim 1, **characterised in that** the casing (7) is formed like a bag in such a manner that it encloses the distal end of the connecting piece (1).

3. The anchor sleeve according to claim 2, **characterised in that** the casing (7) extends beyond the distal end of the connecting piece (1).

4. The anchor sleeve according to claim 2 or 3, **characterised in that** the connecting piece (1) has an end opening (5) at its distal end.

5. The anchor sleeve according to claim 1, **characterised in that** in the area of its proximal end the casing (7) sealingly connects to the outside of the connecting piece (1).

6. The anchor sleeve according to claim 5, **characterised in that** the connecting piece (1) has a tip (15) at its distal end.

7. The anchor sleeve according to any one of claims 1 to 6, **characterised in that** the connecting piece (1) is made of plastics, ceramics or metal, such as steel or titanium.

8. The anchor sleeve according to any one of claims 1 to 7, **characterised in that** the casing (7) is impermeable.

9. The anchor sleeve according to any one of claims 1 to 7, **characterised in that** the casing (7) has openings distributed over its surface.

10. The anchor sleeve according to any one of claims 1 to 9, **characterised in that** the casing (7) is made of an inelastic material.

11. The anchor sleeve according to any one of claims 1 to 9, **characterised in that** the casing (7) is made of an elastic material.

12. The anchor sleeve according to any one of claims 1 to 11, **characterised in that** the casing (7) is made at least in part of a cloth or a fabric.

13. The anchor sleeve according to any one of claims 1 to 12, **characterised in that** the casing (7) is made at least in part of a foil.

14. The anchor sleeve according to any one of claims 1 to 13, **characterised in that** the casing (7) contains mineral fibres.

## Revendications

1. Douille d'ancrage pour le raccordement à au moins un os ou un fragment (9, 10) d'un os, la douille comprenant un embout (1) avec un canal de distribution (3) émanant d'une ouverture d'entrée (2) au niveau d'une extrémité proximale, s'étendant dans une direction axiale vers une extrémité distale et ayant plusieurs ouvertures de sortie disposées à l'extérieur de l'embout (1), et avec une enveloppe (7) en matériau flexible qui se connecte de manière étanche à l'extérieur de l'embout (1) et enferme la partie de l'extérieur de l'embout portant les ouvertures de sortie, **caractérisée en ce que** les ouvertures de sortie s'étendent sur la longueur de l'embout ou sont réparties sur la longueur de l'embout.

2. Douille d'ancrage selon la revendication 1, **caractérisée en ce que** l'enveloppe (7) est configurée en forme de sac de telle manière qu'elle enferme l'extrémité distale de l'embout (1).

3. Douille d'ancrage selon la revendication 2, **caractérisée en ce que** l'enveloppe (7) s'étend au-delà de l'extrémité distale de l'embout (1).

4. Douille d'ancrage selon la revendication 2 ou 3, **caractérisée en ce que** l'embout (1) présente une ouverture d'extrémité (5) au niveau de son extrémité distale.

5. Douille d'ancrage selon la revendication 1, **caractérisée en ce que** l'enveloppe (7) se connecte, dans la région de son extrémité distale, à l'extérieur de l'embout (1) de manière étanche.

6. Douille d'ancrage selon la revendication 5, **caractérisée en ce que** l'embout (1) présente une pointe (15) au niveau de son extrémité distale.

7. Douille d'ancrage selon l'une quelconque des revendications 1 à 6, **caractérisée en ce que** l'embout (1) est réalisé en matière plastique, en céramique ou en métal, tel que l'acier ou le titane.

8. Douille d'ancrage selon l'une quelconque des revendications 1 à 7, **caractérisée en ce que** l'enveloppe (7) est imperméable.

9. Douille d'ancrage selon l'une quelconque des revendications 1 à 7, **caractérisée en ce que** l'enveloppe (7) présente des ouvertures réparties sur sa surface.

10. Douille d'ancrage selon l'une quelconque des revendications 1 à 9, **caractérisée en ce que** l'enveloppe (7) est réalisée en matière inélastique.

11. Douille d'ancrage selon l'une quelconque des revendications 1 à 9, **caractérisée en ce que** l'enveloppe (7) est réalisée en matière élastique.

12. Douille d'ancrage selon l'une quelconque des revendications 1 à 11, **caractérisée en ce que** l'enveloppe (7) est réalisée au moins partiellement en tissu ou tricot.

13. Douille d'ancrage selon l'une quelconque des revendications 1 à 12, **caractérisée en ce que** l'enveloppe (7) est réalisée au moins partiellement par une feuille.

14. Douille d'ancrage selon l'une quelconque des revendications 1 à 13, **caractérisée en ce que** l'enveloppe (7) contient des fibres minérales.
